# EUROPEAN PATENT SPECIFICATION

(11) **EP 2 266 464 B1**
(45) Date of publication and mention of the grant of the patent: **24.07.2013**
(21) Application number: 10006556.4
(22) Date of filing: 23.06.2010
(51) Int. Cl.: A61B 8/08, A61B 5/00

(54) **Ultrasonic diagnosis apparatus and ultrasonic diagnosis support information providing method**
Ultraschalldiagnosevorrichtung und Verfahren zur Bereitstellung von Ultraschalldiagnosehilfsinformationen
Appareil de diagnostic à ultrasons et procédé fournissant des informations sur le support de diagnostic à ultrasons

(30) Priority: 26.06.2009 JP 2009152338; 02.06.2010 JP 2010126589
(43) Date of publication of application: 29.12.2010
(73) Proprietor: Kabushiki Kaisha Toshiba, Minato-ku, Tokyo 105-8001 (JP); Toshiba Medical Systems Corporation, Otawara-shi Tochigi 324-8550 (JP)
(72) Inventor: Okamura, Yoko, Otawara-shi Tochigi 324-8550 (JP); Kamiyama, Naohisa, Otawara-shi Tochigi 324-8550 (JP)
(74) Representative: Kramer - Barske - Schmidtchen

(56) References cited:
- EP-A1- 1 652 477
- EP-A1- 2 138 103
- JP-A- 2004 351 062
- US-A1- 2006 036 172

## Description

### FIELD

Embodiments described herein relate generally to an ultrasonic diagnosis apparatus and an ultrasonic diagnosis support information providing method.

### BACKGROUND

The present invention relates to an ultrasonic diagnosis apparatus and ultrasonic diagnosis support information providing method for image diagnosis of the hardness of a tumorous lesion and the like.

Ultrasonic diagnosis allows to display in real time how the heart beats or the fetus moves, by simply bringing an ultrasonic probe into contact with the body surface. In addition, ultrasonic diagnosis is free from the influences of exposure using X-rays and the like, and hence is high in safety and can be used in an obstetrical section, for medical examination of breast cancers, for home medical care, and the like. An ultrasonic diagnosis apparatus is an apparatus used for such ultrasonic diagnosis. This apparatus is smaller in system size than other diagnosis apparatuses using X-rays, CT, MRI, and the like, and can be moved to the bedside to be easily and conveniently used for examination.

EP 2 138 103 A1 is part of the state of the art in accordance with Art. 54(3) EPC and relates to a technique in a case in which tissue strain imaging (TSI) is applied to elastic imaging to estimate a local hardness distribution from the strain state of a biological soft tissue.

There is available so-called tissue elasticity imaging which images the hardness of a tumor, as one of units for benign/malignant discrimination of tumors which use this ultrasonic diagnosis apparatus. This is a technique of acquiring and visualizing elasticity information by measuring the dynamic responsiveness of tissue from the contracting and stretching motions of a diagnosis region caused by externally applying a dynamic load to the diagnosis region (i.e., compressing and relaxing the diagnosis region). A doctor performs image diagnosis of the degree of deformation and dynamic responsiveness of the diagnosis region by using the images acquired by tissue elasticity imaging.

In the above tissue elasticity imaging, one of the important factors for preferred image diagnosis is to properly compress and relax a diagnosis region.

In conventional tissue elasticity imaging, however, a technician or doctor subjectively determines compression and relaxation periods for a diagnosis region. This leads to a lack of objectivity and may make it impossible to properly compress and relax a diagnosis region.

In some cases, it is possible to acquire data with high reproducibility by properly changing the compression and relaxation periods for a diagnosis region in accordance with a patient. In conventional tissue elasticity imaging, since there is no criterion for proper determination of compression and relaxation periods for a diagnosis region, it is impossible to properly perform tissue elasticity imaging in accordance with the individual differences of patients.

An improved ultrasonic diagnosis apparatus is defined in claim 1 and an improved ultrasonic diagnosis support information providing method is defined in claim 8. Further advantageous embodiments are set forth in the dependent claims.

### BRIEF DESCRIPTION OF THE DRAWINGS

FIG. 1 is a block diagram showing the arrangement of an ultrasonic diagnosis apparatus 1 according to this embodiment;
FIG. 2 is a flowchart showing the operation procedure of this ultrasonic diagnosis apparatus in tissue elasticity imaging using a compression/relaxation support function;
FIG. 3 is a chart for explaining the first example of a reference period decision technique, showing a Doppler waveform associated with a predetermined region of interest in a diagnosis region acquired in step S3;
FIG. 4 is a chart for explaining the fourth example of the reference period decision technique, showing a Doppler waveform associated with a predetermined region of interest in the diagnosis region acquired in step S3;
FIG. 5 is a graph showing temporal changes in compression period or relaxation period;
FIG. 6 is a graph showing an example of the output form of a reference period in tissue elasticity imaging in step S5;
FIG. 7 is a view showing another example of the output form of a reference period in tissue elasticity imaging in step S5;
FIG. 8 is a view showing still another example of the output form of a reference period in tissue elasticity imaging in step S5; and
FIG. 9 is a chart for explaining a modification of the ultrasonic diagnosis apparatus according to this embodiment.

### DETAILED DESCRIPTION

An embodiment of the present invention will be described below with reference to the views of the accompanying drawing. Note that the same reference numerals denote constituent elements having substantially the same functions and arrangements in the following description, and a repetitive description will be made only when required.

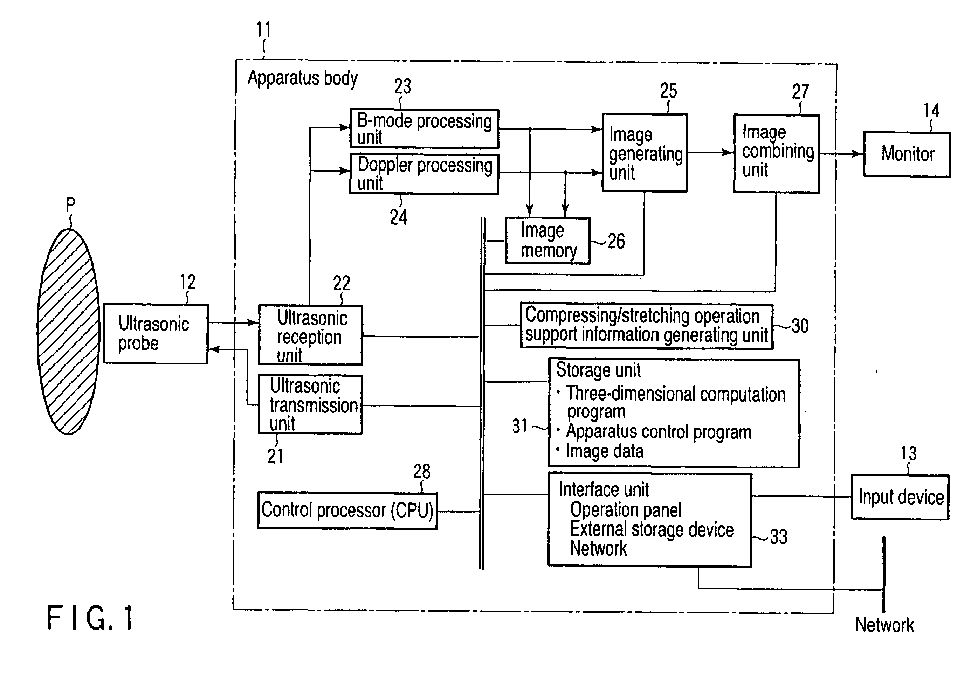

FIG. 1 is a block diagram showing the arrangement of an ultrasonic diagnosis apparatus 1 according to this embodiment. As shown in FIG. 1, the ultrasonic diagnosis apparatus 1 includes an apparatus body 11, and an ultrasonic probe 12, an input device 13, and a monitor 14 which are connected to the apparatus body 11. The apparatus body 11 also includes an ultrasonic transmission unit 21, an ultrasonic reception unit 22, a B-mode processing unit 23, a Doppler processing unit 24, an image generating unit 25, an image memory 26, an image combining unit 27, a control processor (CPU) 28, a compressing/relaxing operation support information generating unit 30, a storage unit 31, and an interface unit 33. The function of each constituent element will be described below.

The ultrasonic probe 12 includes a plurality of piezoelectric transducers which generate ultrasonic waves based on driving signals from the ultrasonic transmission unit 21 and convert reflected waves from an object into electrical signals, a matching layer provided for the piezoelectric transducers, and backing member which prevents ultrasonic waves from propagating backward from the piezoelectric transducers. When the ultrasonic probe 12 transmits an ultrasonic wave to an object P, the transmitted ultrasonic wave is sequentially reflected by a discontinuity surface of acoustic impedance of internal body tissue, and is received as an echo signal by the ultrasonic probe 12. The amplitude of this echo signal depends on an acoustic impedance difference on the discontinuity surface by which the echo signal is reflected. The echo produced when a transmitted ultrasonic pulse is reflected by a moving blood flow or tissue is subjected to a frequency shift depending on the velocity component of the moving body in the ultrasonic transmission direction due to a Doppler effect.

The input device 13 is connected to the apparatus body 11 and includes various types of switches, buttons, a trackball, a mouse, and a keyboard which are used to input, to the apparatus body 11, various types of instructions, conditions, an instruction to set a region of interest (ROI), various types of image quality condition setting instructions, and the like from an operator. When, for example, the operator operates the end button or FREEZE button of the input device 13, the transmission/reception of ultrasonic waves is terminated, and the ultrasonic diagnosis apparatus is set in a temporary stop state.

The monitor 14 displays morphological information and blood flow information in the living body as images based on video signals from the image generating unit 25. The monitor 14 also displays information informing the operator of a reference period (to be described later) in a predetermined form.

The ultrasonic transmission unit 21 includes a trigger generating circuit, delay circuit, and pulser circuit (none of which are shown). The pulser circuit repetitively generates rate pulses for the formation of transmission ultrasonic waves at a predetermined rate frequency fr Hz (period: 1/fr sec). The delay circuit gives each rate pulse a delay time necessary to focus an ultrasonic wave into a beam and determine transmission directivity for each channel. The trigger generating circuit applies a driving pulse to the probe 12 at the timing based on this rate pulse.

The ultrasonic transmission unit 21 has a function of instantly changing a transmission frequency, transmission driving voltage, or the like to execute a predetermined scan sequence in accordance with an instruction from the control processor 28. In particular, the function of changing a transmission driving voltage is implemented by linear amplifier type transmission circuit capable of instantly switching its value or a mechanism of electrically switching a plurality of power supply units.

The ultrasonic reception unit 22 includes an amplifier circuit, A/D converter, and adder (none of which are shown). The amplifier circuit amplifies an echo signal received via the probe 12 for each channel. The A/D converter gives the amplified echo signals delay times necessary to determine reception directivities. The adder then performs addition processing for the signals. With this addition, a reflection component is enhanced from a direction corresponding to the reception directivity of the echo signal to form a composite beam for ultrasonic transmission/reception in accordance with reception directivity and transmission directivity.

The B-mode processing unit 23 receives an echo signal from the ultrasonic reception unit 22, and performs logarithmic amplification, envelope detection processing, and the like for the signal to generate B-mode image data whose signal intensity is expressed by a luminance level.

The Doppler processing unit 24 frequency-analyzes velocity information from the echo signal received from the ultrasonic reception unit 22, extracts a blood flow or tissue owing to a Doppler effect and a contrast medium echo component, and obtains blood information such as mean velocities, variances, powers, and the like at multiple points. The obtained blood flow information is sent to the image generating circuit 25, and is displayed in color as a mean velocity image, a variance image, a power image, and a combined image thereof on the monitor 14.

In addition to the operation described above, the image generating circuit 25 converts the scanning line signal string for ultrasonic scanning operation into a scanning line signal string in a general video format typified by a TV format, thereby generating an ultrasonic diagnosis image as a display image. The image generating unit 25 includes a memory to store image data, and can perform three-dimensional image reconstruction processing and the like. This unit allows the operator to call up an image recorded during examination after diagnosis. Note that data before it is input to the image generating unit 25 is sometimes called "raw data".

The image memory 26 is a memory to store, for example, ultrasonic images corresponding to a plurality of frames immediately before a freeze. Continuously displaying (cine-displaying) images stored in the image memory 26 can display an ultrasonic moving image.

The image combining unit 27 combines the image received from the image generating unit 25 with character information of various types of parameters, scale marks, and the like, and outputs the resultant signal as a video signal to the monitor 14. The image combining unit 27 also stores a three-dimensional reconstruction program, an image processing program according to the present invention, and the like. These programs are activated in response to instructions from the operator and the like.

The control processor 28 is a control unit which has the function of an information processing apparatus (computer) and controls the operation of the main body of this ultrasonic diagnosis apparatus. The control processor 28 reads out a control program for executing image generation/display and the like from an internal storage unit 29, expands the program in the memory of the internal storage unit 29, and executes computation, control, and the like associated with each type of processing.

The compressing/relaxing operation support information generating unit 30 executes processing based on a compression/relaxation support function (to be described later). That is, the compressing/relaxing operation support information generating unit 30 generates a reference period for the objective determination of a compression period or relaxation period for a diagnosis region based on an actually measured change in the velocity of the diagnosis region in compressing/relaxing operation.

The storage unit 31 stores transmission/reception conditions, control programs for executing image generation and display processing, diagnosis information (patient ID, findings by doctors, and the like), a diagnosis protocol, a body mark generation program, and other data. The storage unit 31 is also used to store images in the image memory 26, as needed. It is possible to transfer data in the storage unit 31 to an external peripheral device via the interface unit 33.

The interface unit 33 is an interface associated with the input device 13, a network, and a new external storage device (not shown). The interface unit 33 can transfer data such as ultrasonic images, analysis results, and the like obtained by this apparatus to another apparatus via a network.

### (Compression/Relaxation Support Function)

The compression/relaxation support function of the ultrasonic diagnosis apparatus 1 will be described next. This function is configured to support the operation of applying a dynamic load in tissue elasticity imaging and improve the quality of image diagnosis. This function is obtained by generating and providing information indicating a period (reference period) as a reference for the objective determination of a period (compression period) in which the operator compresses a diagnosis region or a period (relaxation period) in which the operator relaxes the diagnosis region, by using ultrasonic images, in a case in which the operator applies a dynamic load to the diagnosis region (i.e., compresses/relaxes the diagnosis region) in tissue elasticity imaging.

For the sake of a concrete description, this embodiment exemplifies a case in which a diagnosis region is a breast. However, the present invention is not limited to this, and the compression/relaxation support function can be applied to other regions, as diagnosis regions, including, for example, the liver, pancreas, thyroid glands, and prostate glands.

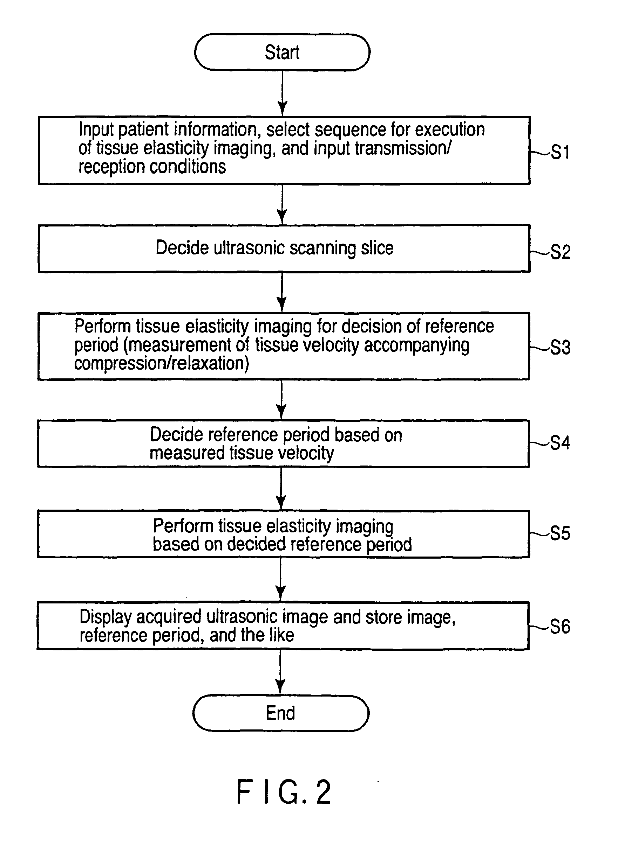

FIG. 2 is a flowchart showing the operation procedure of this ultrasonic diagnosis apparatus in tissue elasticity imaging using this compression/relaxation support function. As shown in FIG. 2, first of all, for example, the operator inputs patient information, selects an imaging sequence for the execution of tissue elasticity imaging, and inputs transmission/reception conditions via the input device 13 (step S1). The operator acquires an ultrasonic image in real time, adjusts the ultrasonic probe 12 to a proper position while observing the image, and decides a slice (scanning slice) on which ultrasonic scanning is performed to acquire an ultrasonic image associated with the diagnosis region (step S2).

This apparatus then executes tissue elasticity imaging for the decision of a reference period (step S3). That is, the operator scans the scanning slice with ultrasonic waves in, for example, the tissue Doppler mode, while repeatedly compressing and relaxing the diagnosis region, and acquires an ultrasonic image of the scanning slice. This makes it possible to measure the contraction velocity (or shrinkage velocity)/stretching velocity (or expansion velocity) of the tissue of the diagnosis region accompanying compression/relaxation.

For the sake of a concrete description, this embodiment has exemplified the case in which the tissue Doppler mode is used to acquire an ultrasonic image of a scanning slice. However, the present invention is not limited to this. For example, the embodiment may acquire ultrasonic image data of a scanning slice or a volume including the scanning slice in the B mode, calculate the moving distance of the diagnosis region between frames or volumes by tracking processing, and measure the contraction velocity/stretching velocity of the tissue of the diagnosis region accompanying compression/relaxation by using the measurement result and the frame rate or the volume rate.

The compressing/relaxing operation support information generating unit 30 then decides a reference period to be referred to when the diagnosis region is compressed and relaxed, based on the measured contraction velocity/stretching velocity of the tissue of the diagnosis region accompanying compression/relaxation (step S4). The reference period decision technique to be used is not specifically limited. The following four examples of this technique are typical examples.

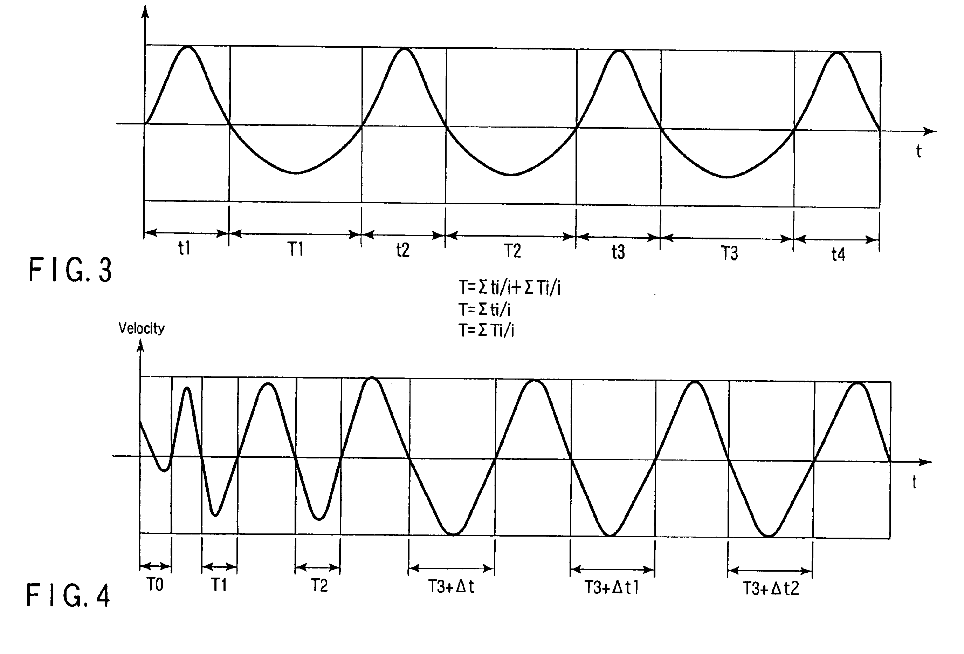

FIG. 3 is a chart for explaining the first example of the reference period decision technique, showing a Doppler waveform associated with a predetermined region of interest in a diagnosis region which is acquired in step S3. Referring to FIGS. 3, 4, and 9, the direction of contraction due to compression is defined as the positive direction of the velocity, and the direction of stretching due to relax is defined as the negative direction of the velocity. The compressing/relaxing operation support information generating unit 30 specifies each period (compression period ti) corresponding to the compression of a diagnosis region and each period (relaxation period Ti) corresponding to the relax of the diagnosis region in a Doppler waveform based on the time phase where the velocity becomes 0 and the velocity directions before and after the time phase. The compressing/relaxing operation support information generating unit 30 calculates the average value (Σti/i) of a plurality of specified compression periods, and decides the result as a reference period.

The second example is a technique of calculating the average value (ΣTi/i) of a plurality of specified relaxation periods and deciding the result as a reference period. It is generally said that a relaxation period reflects the average elastic force of a target tissue more than a compression period. It can therefore be said that this example of using the average value of relaxation periods as a reference period is a more preferred embodiment.

The third example is a technique of calculating an average value (ΣTi/i + ΣTi/i) of both a plurality of specified compression periods and a plurality of specified relaxation periods and deciding the result as a reference period. This example is a preferred example when it is desired to reflect both compression periods and relaxation periods.

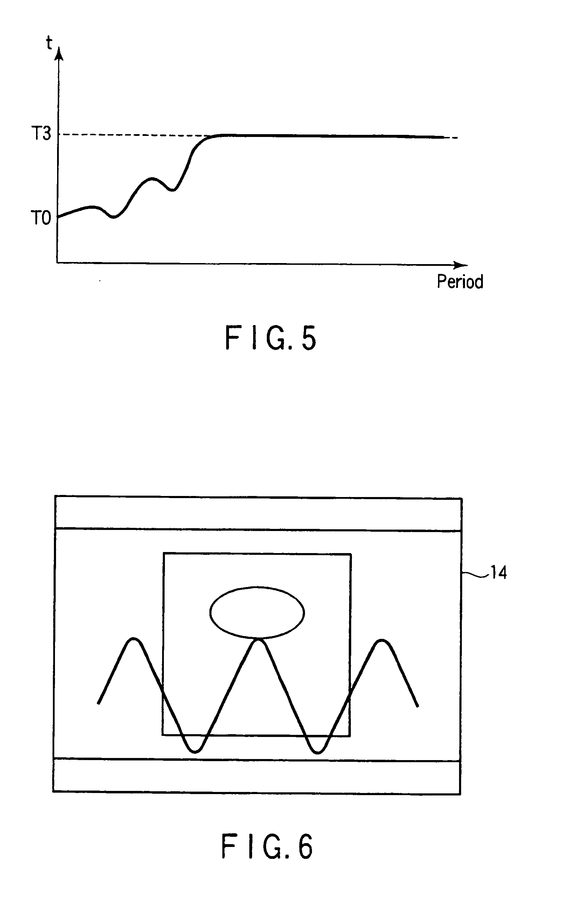

FIG. 4 is a chart for explaining the fourth example of the reference period decision technique, showing a Doppler waveform associated with a predetermined region of interest in a diagnosis region which is acquired in step S3. FIG. 5 is a graph showing temporal changes in compression period or relaxation period. As shown in FIG. 4, in many cases, compression periods and relaxation periods vary in an early interval of compression/relaxation and become stable after repetitive compression/relaxation to approach a predetermined time (T3 in FIGS. 4 and 5), as shown in, for example, FIG. 5. The fourth example is therefore a technique of calculating the asymptotic value of one of a compression period and a relaxation period or the asymptotic value based on both a compression period and a relaxation period, approximating at least one of the compression period and the relaxation period by using the calculated asymptotic value, and setting the result as a reference period. Since a relaxation period reflects the average elastic force of target tissue more than a compression period, it is also preferable for this example to use the asymptotic value of the relaxation period.

This apparatus then executes tissue elasticity imaging based on the decided reference period (step S5). That is, the control processor 28 outputs the decided reference period in a predetermined form to inform the operator of it. The output form of the reference period to be used is not specifically limited. The following four examples are typical examples. It is possible to use the following four examples singly or in combination with each other to effectively inform the operator of a reference period.

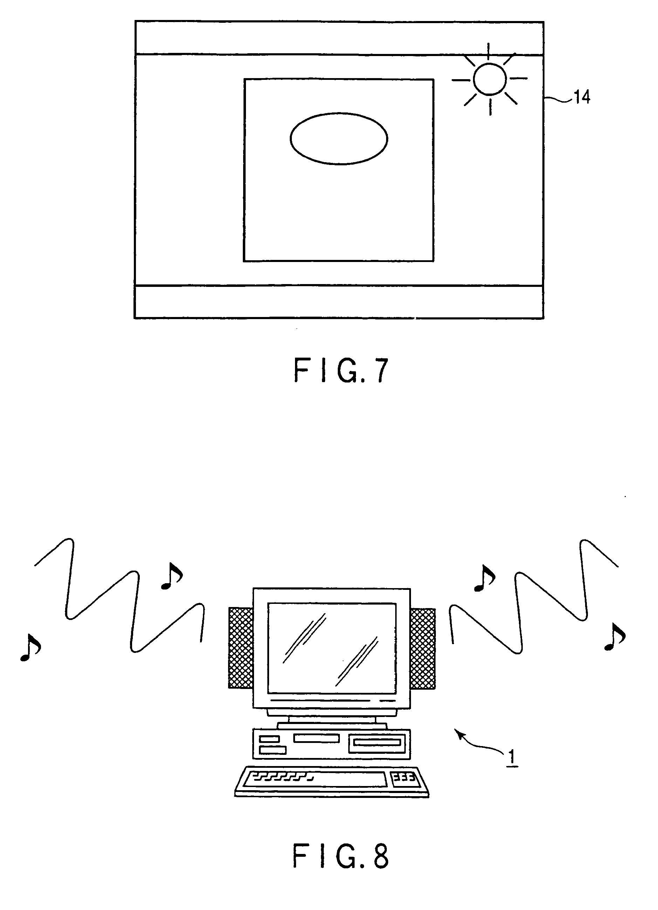

In the first example, the control processor 28 can output a waveform in the form of a video to inform the operator of a compression period (relaxation period) or both a compression period and a relaxation period, as shown in FIG. 6, to allow the operator to visually determine a reference period. In the second example, the control processor 28 can output corresponding information by turning on light or using blinking light only during a compression period (or only a relaxation period), as shown in, for example, FIG. 7, to allow the operator to visually determine a reference period in the same manner as described above. In the third example, the control processor 28 can output a sound to inform the operator that a compression period (or a relaxation period) is underway, as shown in, for example, FIG. 8, to allow the operator to aurally determine a reference period. In the fourth example, the control processor 28 may output, for example, vibrations to inform the operator that a compression period (a relaxation period) is underway or vibrations to inform the operator of a compression pace (relax pace) through the contact surface between the ultrasonic probe 12 and the hand of the operator or a dedicated vibrator attached to the operator to allow the operator to tactually determine a reference period.

The operator acquires an ultrasonic image of a scanning slice by scanning the scanning slice with ultrasonic waves in the tissue Doppler mode while repeatedly compressing and relaxing the diagnosis region based on the output reference period. With this operation, the operator repeatedly compresses and relaxes the diagnosis region based on an objective index called a reference period, thus executing tissue elasticity imaging associated with the diagnosis region.

The control processor 28 displays the ultrasonic image acquired in step S5 on the monitor in real time, and manages and stores it for each series in the storage unit 31, as needed. The control processor 28 stores the reference period which has been referred to in tissue elasticity imaging in step S5 in the storage unit 31 in correspondence with patient information or an image ID. It is possible to reproduce and use the stored reference period when, for example, performing tissue elasticity imaging for the same patient at a later date.

The above arrangement can obtain the following effects.

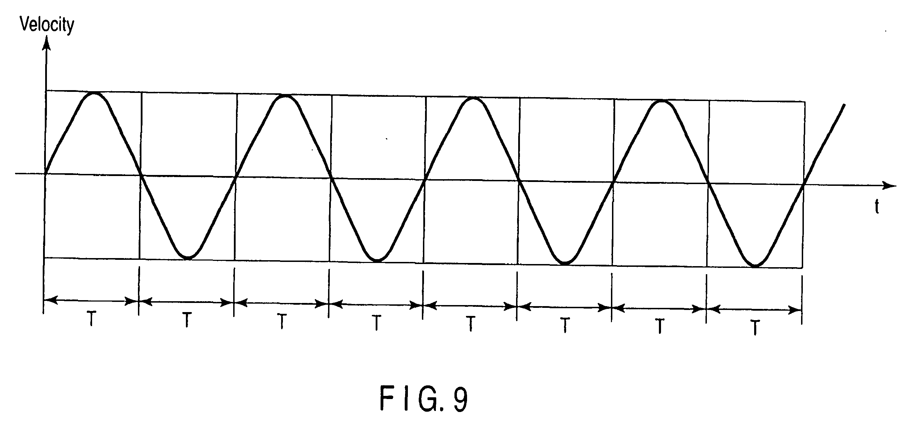

According to this ultrasonic diagnosis apparatus, when the operator compresses and relaxes a diagnosis region by applying a dynamic load to it in tissue elasticity imaging, the apparatus measures changes in the velocity of the diagnosis region in actual compressing/relaxing operation, and generates and displays information informing the operator of a reference period for the objective determination of a compression period and a relaxation period for the diagnosis region based on the measurement result. The operator can therefore implement stable tissue elasticity imaging free from variations by compressing and relaxing the diagnosis region in accordance with the information informing the operator of the reference period which is, for example, displayed. It is said to be ideal to compress and relax a diagnosis region in tissue elasticity imaging, in particular, in such a manner that an area S1 (contraction distance) and an area S2 (stretching distance) each defined by the Doppler waveform of the tissue and the time axis become equal to each other (see, for example, FIG. 9). The operator can implement ideal tissue elasticity imaging easily and stably by compressing and relaxing a diagnosis region in accordance with the reference period provided by this ultrasonic diagnosis apparatus.

This ultrasonic diagnosis apparatus generates information informing the operator of a reference period to be provided, based on the tissue velocity actually measured by compressing and relaxing the diagnosis region of the object. It is therefore possible to generate and change a suitable compression period and relaxation period for a diagnosis region, as needed, in accordance with a patient based on objective information. This can implement tissue elasticity imaging with high reproducibility and quality without any load.

This ultrasonic diagnosis apparatus outputs information informing the operator of a reference period to be provided in the form that allows the operator to comprehend the information visually, aurally, or tactually. The operator can therefore easily and quickly comprehend a compression period and a relaxation period for a diagnosis region.

Note that the present invention is not limited to the above embodiment, and constituent elements can be variously modified and embodied at the execution stage within the scope of the invention. The following are concrete modifications.
(1) Each function associated with each embodiment can also be implemented by installing programs for executing the corresponding processing in a computer such as a workstation and mapping them in a memory. In this case, the programs which can cause the computer to execute the corresponding techniques can be distributed by being stored in recording media such as magnetic disks (floppy^{®} disks, hard disks, and the like), optical disks (CD-ROMs, DVDs, and the like), and semiconductor memories.
(2) The above embodiment is configured to measure changes in the velocity of a diagnosis region when it is actually compressed and relaxed and decide a reference period based on the measurement result. However, the present invention is not limited to this. For example, as shown in FIG. 9, it is possible to decide, as a reference period, an average relaxation period, an average compression period, or the average period of relax and compression periods prepared in advance from clinical experiences. This arrangement, can save tissue elasticity imaging for the decision of a reference period (i.e., the processing in step S3 in FIG. 2), and has the effect of shortening the time required for tissue elasticity imaging in addition to the effects described in the above embodiment.

While certain embodiments have been described, these embodiments have been presented by way of example only, and are not intended to limit the scope of the invention. Indeed, the novel embodiments described herein may be embodied in a variety of other forms; furthermore, various omissions, substitutions and changes in the form of the embodiments described herein may be made without departing from the invention. The accompanying claims and their equivalents are intended to cover such forms or modifications as would fall within the scope of the invention.

It is explicitly stated that all features disclosed in the description and/or the claims are intended to be disclosed separately and independently from each other for the purpose of original disclosure as well as for the purpose of restricting the claimed invention independent of the composition of the features in the embodiments. It is explicitly stated that all value ranges or indications of groups of entities disclose every possible intermediate value or intermediate entity for the purpose of original disclosure as well as for the purpose of restricting the invention, in particular as limits of value ranges.

## Claims

1. An ultrasonic diagnosis apparatus, comprising:
an image data acquisition unit (21, 22, 23, 24) configured to acquire ultrasonic image data corresponding to each time phase in a first interval including at least one contraction and one stretching by scanning a diagnosis region of an object which repeats a contracting motion and a stretching motion, with an ultrasonic wave, accompanying application of a dynamic load including repetitive contraction and relaxation, over the first interval;
a velocity information generating unit (30) configured to generate a variation with time of velocity of the diagnosis region associated with the contracting motion and the stretching motion by using the ultrasonic image data corresponding to each time phase in the first interval;
a reference period decision unit (30) configured to calculate an average period or an asymptotic value of at least one of the contracting motion and the stretching motion, and, based on the calculation, decide a reference period as a reference for a period of the dynamic load; and
an output unit (14) configured to output the reference period in a predetermined form to be used as the reference for the period of the dynamic load.

2. The apparatus according to claim 1, **characterized in that** the reference period decision unit (30) is adapted to determine at least a plurality of relax intervals with the dynamic load based on a time phase where a velocity included in the velocity information becomes zero and a velocity direction, and to decide the reference period by using the plurality of determined relax intervals.

3. The apparatus according to claim 1 or 2, **characterized in that** the reference period decision unit (30) is adapted to determine at least a plurality of compression intervals with the dynamic load based on a time phase where a velocity included in the velocity information becomes zero and a velocity direction, and to decide the reference period by using the plurality of determined compression intervals and/or the plurality of determined relax intervals.

4. The apparatus according to any of claims 1 to 3, **characterized in that** the reference period decision unit (30) is adapted to decide the reference period by averaging processing or approximation processing using an asymptotic value.

5. The apparatus according to any of claims 1 to 4, **characterized in that** the output unit (14) is adapted to output the support information in the form of visible information informing that one of a compression period and a relaxation period is underway.

6. The apparatus according to claim 5, **characterized in that** the output unit (14) is adapted to display a waveform indicating at least one of a compression period and a relaxation period as the support information.

7. The apparatus according to any of claims 1 to 6, **characterized in that** the output unit (14) is adapted to output the support information in the form of a sound and/or a vibration informing that one of a compression period and a relaxation period is underway.

8. An ultrasonic diagnosis support information providing method, comprising:
acquiring ultrasonic image data corresponding to each time phase in a first interval including at least one contraction and one stretching by scanning a diagnosis region of an object which repeats a contracting motion and a stretching motion, with an ultrasonic wave, accompanying application of a dynamic load including repetitive contraction and relaxation, over the first interval;
generating a variation with time of velocity of the diagnosis region associated with the contracting motion and the stretching motion by using the ultrasonic image data corresponding to each time phase in the first interval;
calculating an average period or an asymptotic value of at least one of the contracting motion and the stretching motion, and, based on the calculation, deciding a reference period as a reference for a period of the dynamic load; and
outputting the reference period in a predetermined form to be used as the reference for the period of the dynamic load.

9. The method according to claim 8, **characterized by** further comprising determining at least a plurality of relax intervals with the dynamic load based on a time phase where a velocity included in the velocity information becomes zero and a velocity direction, and deciding the reference period by using the plurality of determined relax intervals.

10. The method according to claim 8 or 9, **characterized by** further comprising determining at least a plurality of compression intervals with the dynamic load based on a time phase where a velocity included in the velocity information becomes zero and a velocity direction, and deciding the reference period by using the plurality of determined compression intervals and/or the plurality of determined relax intervals.

11. The method according to any of claims 8 to 10, **characterized in that** the reference period decision unit (30) decides the reference period by averaging processing or approximation processing using an asymptotic value.

12. The method according to any of claims 8 to 11, **characterized in that** the support information is output in the form of visible information informing that one of a compression period and a relaxation period is underway.

13. The method according to claim 12, **characterized in that** a waveform indicating at least one of a compression period and a relaxation period is displayed as the support information.

14. The method according to any of claims 8 to 13, **characterized in that** the support information is output in the form of a sound and/or a vibration informing that one of a compression period and a relaxation period is underway.

## Patentansprüche

1. Ultraschalldiagnosevorrichtung, mit:
einer Bilddatenerfassungseinheit (21, 22, 23, 24), die konfiguriert ist zum Erfassen von Ultraschallbilddaten entsprechend jeder Zeitphase in einem ersten Intervall, das mindestens eine Kontraktion und Streckung enthält, indem eine Diagnoseregion eines Objekts, das eine Kontraktionsbewegung und eine Streckbewegung wiederholt, mit einer Ultraschallwelle zusammen mit einer Anwendung einer dynamischen Last, die eine wiederholte Kontraktion und Entspannung enthält, über das erste Intervall abgetastet wird;
einer Geschwindigkeitsinformationserzeugungseinheit (30), die konfiguriert ist zum Erzeugen einer zeitlichen Änderung der Geschwindigkeit der Diagnoseregion, die zu der Kontraktionsbewegung und Streckbewegung gehört, indem die Ultraschallbilddaten verwendet werden, die jeder Zeitphase in dem ersten Intervall entsprechen;
einer Referenzperiodenentscheidungseinheit (30), die konfiguriert ist zum Berechnen einer Durchschnittsperiode oder eines asymptotischen Werts von mindestens einer von der Kontraktionsbewegung und der Streckbewegung, und eine Referenzperiode als eine Referenz für eine Periode der dynamischen Last basierend auf der Berechnung bestimmt; und
einer Ausgabeeinheit (14), die konfiguriert ist zum Ausgeben der Referenzperiode in einer vorbestimmten zu verwendenden Form, als die Referenz für die Periode der dynamischen Last.

2. Vorrichtung nach Anspruch 1, **dadurch gekennzeichnet, dass** die Referenzperiodenentscheidungseinheit (30) angepasst ist zum Bestimmen von mindestens einer Mehrzahl von Entspannungsintervallen mit der dynamischen Last basierend auf einer Zeitphase, wo eine Geschwindigkeit, die in der Geschwindigkeitsinformation enthalten ist, Null wird, und einer Geschwindigkeitsrichtung, und zum Bestimmen der Referenzperiode durch Verwenden der Mehrzahl der bestimmten Entspannungsintervalle.

3. Vorrichtung nach Anspruch 1 oder 2, **dadurch gekennzeichnet, dass** die Referenzperiodenentscheidungseinheit (30) angepasst ist zum Bestimmen von mindestens einer Mehrzahl von Kompressionsintervallen mit der dynamischen Last basierend auf einer Zeitphase, wo eine Geschwindigkeit, die in der Geschwindigkeitsinformation enthalten ist, Null wird, und einer Geschwindigkeitsrichtung, und zum Bestimmen der Referenzperiode, indem die Mehrzahl der bestimmten Kompressionsintervalle und/oder die Mehrzahl der bestimmten Entspannungsintervalle verwendet wird.

4. Vorrichtung nach einem der Ansprüche 1 bis 3, **dadurch gekennzeichnet, dass** die Referenzperiodenentscheidungseinheit (30) angepasst ist zum Entscheiden der Referenzperiode durch eine Mittelwertverarbeitung oder Näherungsverarbeitung unter Verwendung eines asymptotischen Werts.

5. Vorrichtung nach einem der Ansprüche 1 bis 4, **dadurch gekennzeichnet, dass** die Ausgabeeinheit (14) angepasst ist zum Ausgeben der Unterstützungsinformation in der Form von sichtbarer Information, die darüber informiert, dass eine von einer Kompressionsperiode und einer Entspannungsperiode im Gange ist.

6. Vorrichtung nach Anspruch 5, **dadurch gekennzeichnet, dass** die Ausgabeeinheit (14) angepasst ist zum Anzeigen einer Wellenform, die mindestens eine von einer Kompressionsperiode und einer Entspannungsperiode als die Unterstützungsinformation angibt.

7. Vorrichtung nach einem der Ansprüche 1 bis 6, **dadurch gekennzeichnet, dass** die Ausgabeeinheit (14) angepasst ist zum Ausgeben der Unterstützungsinformation in der Form eines Geräuschs und/oder einer Vibration, die darüber informiert, dass eine von einer Kompressionsperiode und einer Entspannungsperiode im Gange ist.

8. Ultraschalldiagnoseunterstützungsinformation-Bereitstellungsverfahren, enthaltend:
Erfassen von Ultraschallbilddaten, die jeder Zeitphase in einem ersten Intervall entsprechen, das mindestens eine Kontraktion und eine Streckung enthält, indem eine Diagnoseregion eines Objekts, das eine Kontraktionsbewegung und eine Streckbewegung wiederholt, mit einer Ultraschallwelle zusammen mit einer Anwendung einer dynamischen Last, die eine wiederholte Kontraktion und Entspannung enthält, über das erste Intervall abgetastet wird;
Erzeugen einer zeitlichen Änderung der Geschwindigkeit der Diagnoseregion, die zu der Kontraktionsbewegung und der Streckbewegung gehört, indem die Ultraschallbilddaten verwendet werden, die jeder Zeitphase in dem ersten Intervall entsprechen;
Berechnen einer Durchschnittsperiode oder eines asymptotischen Werts von mindestens einer von der Kontraktionsbewegung und der Streckbewegung, Bestimmen einer Referenzperiode als eine Referenz für eine Periode der dynamischen Last basierend auf der Berechnung; und
Ausgeben der Referenzperiode in einer vorbestimmten Form, die zu verwenden ist, als die Referenz für die Periode der dynamischen Last.

9. Verfahren nach Anspruch 8, **gekennzeichnet durch** ferner enthaltend ein Bestimmen von mindestens einer Mehrzahl von Entspannungsintervallen mit der dynamischen Last basierend auf einer Zeitphase, wo eine Geschwindigkeit, die in der Geschwindigkeitsinformation enthalten ist, Null wird, und einer Geschwindigkeitsrichtung, und Bestimmen der Referenzperiode indem die Mehrzahl der bestimmten Entspannungsintervalle verwendet wird.

10. Verfahren nach Anspruch 8 oder 9, **gekennzeichnet durch** ferner enthaltend ein Bestimmen von mindestens einer Mehrzahl von Kompressionsintervallen mit der dynamischen Last basierend auf einer Zeitphase, wo eine Geschwindigkeit, die in der Geschwindigkeitsinformation enthalten ist, Null wird, und einer Geschwindigkeitsrichtung, und Bestimmen der Referenzperiode, indem die Mehrzahl der bestimmten Kompressionsintervalle und/oder die Mehrzahl der bestimmten Entspannungsintervalle verwendet wird.

11. Verfahren nach einem der Ansprüche 8 bis 10, **dadurch gekennzeichnet, dass** die Referenzperiodenentscheidungseinheit (30) die Referenzperiode bestimmt, durch Durchschnittsverarbeitung oder Näherungsverarbeitung unter Verwendung eines asymptotischen Werts.

12. Verfahren nach einem der Ansprüche 8 bis 11, **dadurch gekennzeichnet, dass** die Unterstützungsinformation in der Form von sichtbarer Information ausgegeben wird, die darüber informiert, dass eine von einer Kompressionsperiode und einer Entspannungsperiode im Gange ist.

13. Verfahren nach Anspruch 12, **dadurch gekennzeichnet, dass** eine Wellenform, die mindestens eine von einer Kompressionsperiode und einer Entspannungsperiode angibt, als die Unterstützungsinformation angezeigt wird.

14. Verfahren nach einem der Ansprüche 8 bis 13, **dadurch gekennzeichnet, dass** die Unterstützungsinformation in der Form eines Geräusches und/oder einer Vibration ausgegeben wird, die darüber informiert, dass eine von einer Kompressionsperiode und einer Entspannungsperiode im Gange ist.

## Revendications

1. Appareil de diagnostic ultrasonore, comprenant :
une unité d'acquisition de données d'images (21, 22, 23, 24) configurée pour acquérir des données d'images ultrasonores correspondant à chaque phase temporelle dans un premier intervalle comprenant au moins une contraction et un étirement par balayage d'une région de diagnostic d'un objet qui répète un mouvement de contraction et un mouvement d'étirement, avec une onde ultrasonore, accompagnant l'application d'une charge dynamique comprenant une contraction et une relaxation répétitives, pendant le premier intervalle ;
une unité de génération d'informations concernant la vitesse (30) configurée pour générer une variation dans le temps de la vitesse de la région de diagnostic associée au mouvement de contraction et au mouvement d'étirement en utilisant les données d'images ultrasonores correspondant à chaque phase temporelle dans le premier intervalle ;
une unité de décision de période de référence (30) configurée pour calculer une période moyenne ou une valeur asymptotique d'au moins l'un parmi le mouvement de contraction et le mouvement d'étirement, et, en se basant sur le calcul, décider d'une période de référence en tant que référence pour une période de la charge dynamique ; et
une unité de sortie (14) configurée pour sortir la période de référence sous une forme prédéterminée à utiliser en tant que référence pour la période de la charge dynamique.

2. Appareil selon la revendication 1, **caractérisé en ce que** l'unité de décision de période de référence (30) est adaptée pour déterminer au moins une pluralité d'intervalles de relaxation avec la charge dynamique en se basant sur une phase temporelle où une vitesse incluse dans les informations concernant la vitesse devient égale à zéro et une direction de la vitesse, et pour décider de la période de référence en utilisant la pluralité d'intervalles de relaxation déterminés.

3. Appareil selon la revendication 1 ou 2, **caractérisé en ce que** l'unité de décision de période de référence (30) est adaptée pour déterminer au moins une pluralité d'intervalles de compression avec la charge dynamique en se basant sur une phase temporelle où une vitesse incluse dans les informations concernant la vitesse devient égale à zéro et une direction de la vitesse, et pour décider de la période de référence en utilisant la pluralité d'intervalles de compression déterminés et/ou la pluralité d'intervalles de relaxation déterminés.

4. Appareil selon l'une quelconque des revendications 1 à 3, **caractérisé en ce que** l'unité de décision de période de référence (30) est adaptée pour décider de la période de référence par l'établissement d'une moyenne ou l'établissement d'une approximation en utilisant une valeur asymptotique.

5. Appareil selon l'une quelconque des revendications 1 à 4, **caractérisé en ce que** l'unité de sortie (14) est adaptée pour sortir les informations de support sous la forme d'informations visibles informant que l'une parmi une période de compression et une période de relaxation est en cours.

6. Appareil selon la revendication 5, **caractérisé en ce que** l'unité de sortie (14) est adaptée pour afficher une forme d'onde indiquant au moins l'une parmi une période de compression et une période de relaxation en tant qu'informations de support.

7. Appareil selon l'une quelconque des revendications 1 à 6, **caractérisé en ce que** l'unité de sortie (14) est adaptée pour sortir les informations de support sous la forme d'un son et/ou d'une vibration informant que l'une parmi une période de compression et une période de relaxation est en cours.

8. Procédé fournissant des informations de support de diagnostic ultrasonore, comprenant :
l'acquisition de données d'images ultrasonores correspondant à chaque phase temporelle dans un premier intervalle comprenant au moins une contraction et un étirement par balayage d'une région de diagnostic d'un objet qui répète un mouvement de contraction et un mouvement d'étirement, avec une onde ultrasonore, accompagnant l'application d'une charge dynamique comprenant une contraction et une relaxation répétitives, pendant le premier intervalle ;
la génération d'une variation dans le temps de la vitesse de la région de diagnostic associée au mouvement de contraction et au mouvement d'étirement en utilisant les données d'images ultrasonores correspondant à chaque phase temporelle dans le premier intervalle ;
le calcul d'une période moyenne ou d'une valeur asymptotique d'au moins l'un parmi le mouvement de contraction et le mouvement d'étirement, et, en se basant sur le calcul, la décision d'une période de référence en tant que référence pour une période de la charge dynamique ; et
la sortie de la période de référence sous une forme prédéterminée à utiliser en tant que référence pour la période de la charge dynamique.

9. Procédé selon la revendication 8, **caractérisé en ce qu'**il comprend en outre la détermination d'au moins une pluralité d'intervalles de relaxation avec la charge dynamique en se basant sur une phase temporelle où une vitesse incluse dans les informations concernant la vitesse devient égale à zéro et une direction de la vitesse, et la décision de la période de référence en utilisant la pluralité d'intervalles de relaxation déterminés.

10. Procédé selon la revendication 8 ou 9, **caractérisé en ce qu'**il comprend en outre la détermination d'au moins une pluralité d'intervalles de compression avec la charge dynamique en se basant sur une phase temporelle où une vitesse incluse dans les informations concernant la vitesse devient égale à zéro et une direction de la vitesse, et la décision de la période de référence en utilisant la pluralité d'intervalles de compression déterminés et/ou la pluralité d'intervalles de relaxation déterminés.

11. Procédé selon l'une quelconque des revendications 8 à 10, **caractérisé en ce que** l'unité de décision de période de référence (30) décide de la période de référence par l'établissement d'une moyenne ou l'établissement d'une approximation en utilisant une valeur asymptotique.

12. Procédé selon l'une quelconque des revendications 8 à 11, **caractérisé en ce que** les informations de support sont sorties sous la forme d'informations visibles informant que l'une parmi une période de compression et une période de relaxation est en cours.

13. Procédé selon la revendication 12, **caractérisé en ce qu'**une forme d'onde indiquant qu'au moins l'une parmi une période de compression et une période de relaxation est affichée en tant qu'informations de support.

14. Procédé selon l'une quelconque des revendications 8 à 13, **caractérisé en ce que** les informations de support sont sorties sous la forme d'un son et/ou d'une vibration informant que l'une parmi une période de compression et une période de relaxation est en cours.
